Europäisches Patentamt

European Patent Office

Office européen des brevets

(11) Veröffentlichungsnummer: **0 377 147 B1**

(19)

(12) **EUROPÄISCHE PATENTSCHRIFT**

(45) Veröffentlichungstag der Patentschrift: **11.05.94**

(21) Anmeldenummer: **89123086.4**

(22) Anmeldetag: **14.12.89**

(51) Int. Cl.5: **A61L 15/16**, A61K 9/70, A61M 37/00

(54) Transdermales therapeutisches System mit Physostigmin als wirksamem Bestandteil und Verfahren zu seiner Herstellung.

(30) Priorität: **22.12.88 DE 3843238**

(43) Veröffentlichungstag der Anmeldung:
**11.07.90 Patentblatt 90/28**

(45) Bekanntmachung des Hinweises auf die
Patenterteilung:
**11.05.94 Patentblatt 94/19**

(84) Benannte Vertragsstaaten:
**AT BE CH DE ES FR GB GR IT LI LU NL SE**

(56) Entgegenhaltungen:
EP-A- 0 156 080          EP-A- 0 261 402
EP-A- 0 305 756          DE-A- 3 528 979
DE-A- 3 606 892          FR-A- 2 497 457

ADV. BEHAV. BIOL., Band 29 (Alzheimer's Parkinson's Dis.), 1986, Seiten 557-573; D. LEVY et al.: "A novel transdermal therapeutic system as a potential treatment for Alzheimer's disease"

(73) Patentinhaber: **LTS Lohmann Therapie-Systeme GmbH & Co. KG**
**Postfach 23 43**
**D-56513 Neuwied(DE)**

Patentinhaber: **Klinge Pharma GmbH**
**Berg-am-Laim-Strasse 129**
**D-81673 München(DE)**

(72) Erfinder: **Hille, Thomas, Dr.**
**Reckstrasse 17**
**D-5450 Neuwied 1(DE)**
Erfinder: **Hoffmann, Hans-Rainer, Dr.**
**Burghofstrasse 123**
**D-5450 Neuwied 22(DE)**
Erfinder: **Huber, Hans-Joachim, Dr.**
**Ramoltstrasse 28**
**D-8000 München 83(DE)**
Erfinder: **Knoch, Axel, Dr.**
**Neumarkter Strasse 86D**
**D-8000 München 80(DE)**
Erfinder: **Schneider, Gerhard, Dr.**
**Finkenstrasse 27**
**D-8011 Baldham(DE)**
Erfinder: **Stanislaus, Fritz, Dr.**
**Halserspitzstrasse 12**
**D-8000 München 80(DE)**

EP 0 377 147 B1

EP 0 377 147 B1

(74) Vertreter: **Flaccus, Rolf-Dieter, Dr.**
**Patentanwalt**
**Sperlingsweg 32**
**D-50389 Wesseling (DE)**

## Beschreibung

Die Erfindung betrifft ein transdermales therapeutisches System, das Physostigmin als wirksamen Bestandteil enthält und ein Verfahren zu seiner Herstellung.

Die Applikation von Physostigmin zur Behandlung der Alzheimer Krankheit ist in der Literatur beschrieben, wobei die Wirksamkeit der Substanz von verschiedenen Autoren unterschiedlich beurteilt wird. Da das Alkaloid einen hohen first pass effect besitzt - die Bioverfügbarkeit des Physostigmins nach oraler Gabe liegt bei 5 % - müssen die abweichenden Ergebnisse auf Varianten der Applikation zurückgeführt werden.

In der DE-OS 35 28 979 wird eine Zusammensetzung beschrieben, die neben Physostigmin eine Carbonsäure mittlerer Kettenlänge enthält, diese Zusammensetzung kann auf einer Bandage, Einlage oder Kompresse getragen werden, die mittels Verband aufgebracht wird. Diese Applikationsart stellt an sich kein therapeutisches System dar, weshalb vorgesehen ist, die Bandage, Kompresse oder Einlage mit einer Reservoir-Innenschicht, einer undurchlässige Schutzsperrfolie oder einen undurchlässigen Schutzfilm zu versehen und zwischen Reservoir und der Haut eine nicht näher beschriebene Diffusionssteuermembran anzubringen. Weder die Diffusionssteuermembran, noch die Schutzfolien sind näher beschrieben. Die Carbonsäuren werden ausdrücklich als ein wirksames Transportvehikel für die Verabreichung des Arzneimittels durch die Haut bezeichnet, das sonst nicht durch die Hautbarriere hindurchtreten könnte bezeichnet. Diese Aussage ist wissenschaftlich nicht haltbar.

In der DE-PS 36 06 892 wird eine retardierte Applikation von Physostigmin u.a. Wirkstoffen beschrieben, die transdermal erfolgen kann. Eine spezielle Formulierung wird nicht offenbart, vielmehr wird auf eine bereits beschriebene Formulierung (US-PS 3,921,363) verwiesen.

Neben den nur vagen Ausführungen der transdermalen therapeutischesn Systeme wird in keiner der beiden vorstehend erwähnten Offenlegungsschriften auf die Instabilität von Physostigmin eingegangen, die schon früh erkannt wurde (Eber, W., Pharmaz. Ztg. 37, 483 (1988); Herzig, J. Mayer, H., ; Mh. Chem. 18, 379 (1897); Herzig, J. Lieb, H., ebenda 39, 285 (1918); Solvay, A.A., J. chem. Soc. (London) 101, 978 (1912); Instabilität aufgrund einer raschen Zersetzung setzt dem Einsatz des Physostigmins in der Pharmazie enge Grenzen.

Aufgabe der Erfindung ist daher die Bereitstellung von Physostigmin oder eins seiner pharmazeutischen verträglichen Salze in Form eines transdermalen therapeutischen Systems, das Physostigmin oder dessen pharmazeutisch verträgliches Salz über eine Zeitraum von 24 Stunden kontrolliert abgibt und gewährleistet, daß das Physostigmin sich während der Lagerung des vorgefertigten transdermalen therapeutischen Systems nicht merklich zersetzt.

Die Lösung der Aufgabe erfolgt durch die Bereitstellung eines transdermalen therapeutischen Systems zur Verabreichung von Physostigmin an die Haut aus einer wirkstoffundurchlässigen Deckschicht (4), einer haftklebenden Reservoirschicht (1) und ggf. einer wiederablösbaren Schutzschicht, dadurch gekennzeichnet, daß die Reservoirschicht eine Matrix (1) ist, die 10 bis 90 Gew.-% eines Polymermaterials, ausgewählt aus den Gruppen, bestehend aus Blockpolymeren auf Basis von Styrol und 1,3-Dienen, Polyisobutylenen, Polymeren auf Acrylat- und/oder Methacrylatbasis, wobei in die Matrix ein hoher Anteil eines basischen Polymers eingearbeitet ist, ggf. Ester hydrierten Kolophoniums, 0 bis 30 Gew.-% Weichmacher auf Basis von Kohlenwasserstoffen und/oder Estern enthält, und daß auf die Matrix ein Wirkstoffdepot (2) aufgebracht ist, welches Physostigmin in hochprozentiger Form, d.h. mindestens 25%-ig in Lösung mit einer mehr als 10 Kohlenstoffatome besitzenden Carbonsäure und somit unmittelbar nach der Fertigung 15 bis 85 Gew.-% einer Physostigminlösung enthält.

Physostigmin wird mindestens 25 %ig in einem Lösemittel bzw. Lösemittelgemisch gelöst, das physiologisch vertretbar sein muß, da damit zu rechnen ist, daß nicht nur Physostimin, sondern auch das Lösungsmittel durch die Matrix migiert. Diese Lösung kann mittels eines Druckverfahrens, wie z.B. in der DE-OS 36 29 304 beschrieben, auf ein vorher auf eine mit einer wirkstoffundurchlässigen, gegebenenfalls wieder ablösbaren Schutzschicht versehenen Matrix aufgebrachten textilen Flächgebilde aufgedruckt werden. Anschließend werden das bedruckte textile Flächengebilde und die Matrix mit einer vorgefertigten ebenfalls wirkstoffundurchlässigen Deckschicht abgedeckt. Mit einem geeigneten Stanzwerkzeug, dessen Fläche rund und deutlich größer ist als die des Vlieses, wird zentrisch ausgestanzt. Anschließend wird der wirkstofffreie Kleberand abgegittert.

Dieses Verfahren ist im Prinzip bekannt und in der DE-OS 36 29 304 beschrieben. Jedoch dient der oben genannten Offenlegungschrift Nicotinbase als Beispielsubstanz. Da Nicotinbase bei Raumtemperatur flüssig ist, konnte das Verfahren nicht einfach adaptiert werden. Vielmehr mußte Phyostigmin hochprozentig, d.h. mindestens 25 %ig in Lösung gebracht werden, da die Einzeldosierung von pulverförmigen Bestandteilen zwar in der Technologie der festen Arzneiformen gängig ist, in der Technologie der halbfesten Arzneiformen aber gänzlich unbekannt ist, da sich diese Frage bislang nicht stellte. Gleichzeitig

EP 0 377 147 B1

muß das Lösemittel noch drei wichtigen Anforderungen genügen:
- Es darf das höchst instabile Physostigmin nicht zersetzen
- Es muß physiologisch unbedenklich sein.
- Es muß ermöglichen, daß das Physostigmin zwar während der Fertigung des Systems ausgesprochen gut, während der Lagerung aber nur wenig löslich ist.

Carbonsäuren, die mehr als 10 Kohlenstoffatomen besitzen, erfüllen die genannten Forderungen. Beim Mischen von Physostigmin mit diesen Verbindungen kommt es sowohl zur Solubilisierung als auch zur Salzbildung. Grundsätzlich sind solche Zubereitungen bei Raumtemperatur aber flüssig.

Die Löslichkeit des Wirkstoffs in der Matrix wird dadurch verringert (wodurch eine ausreichende Freisetzung erzielt wird), daß in die Matrix ein hoher Anteil eines basischen Polymers, beispielsweise ein Copolymerisat mit kationischem Charakter auf Basis von Dimethylaminoethylmethacrylat und anderen neutralen Methacrylsäureestern, eingearbeitet wird. Dadurch kommt es nach Aufbringen der Matrix zwischen der Carbonsäure und dem basischen Polymer zu einer Säure-Base-Reaktion, wodurch die Löslichkeit des Wirkstoffs deutlich verringert wird. Anhand der Freisetzungen der Rezepturbeispiele kann gezeigt werden, daß die Freisetzungsrate mit zunehmendem Anteil von basischem Polymer in der Matrix ansteigt.

Nachfolgend soll der schematische Aufbau des Systems erläutert werden.

Die Deckschicht (4) kann aus flexiblem oder nicht flexiblem Material bestehen und ein oder mehrschichtig ausgestaltet sein. Substanzen, die zu ihrer Herstellung verwendet werden können, sind polymere Substanzen, wie beispielsweise Polyethylen, Polypropylen, Polyethylenterephtalat, Polyamid. Als weitere Materialien können auch Metallfolien, wie Aluminiumfolie, allein oder mit einem polymeren Substrat beschichtet (im letzteren Falle (5)), angewandt werden. Es können auch textile Flächengebilde verwendet werden, wenn die Bestandteile des Reservoirs aufgrund ihrer physikalischen Beschaffenheit durch das Textilmaterial nicht hindurchtreten können. Bei einer bevorzugten Ausführungsform ist die Deckschicht (4) ein Verbundstoff, der dem Laminat Festigkeit verleiht und als Barriere gegen den Verlust von Laminatbestandteilen dient. Daneben sind auch mit Aluminium bedampfte Folien oder Verbundstoffe geeignet.

An die Deckschicht schließt sich die Matrix (1) an. Während der vierzehntägigen Lagerung tritt Physostigmin aus dem Wirkstoffdepot (2) in die Matrix (1) über und sättigt diese, so daß die Matrix zur Reservoirschicht wird. Die Matrix besitzt die Eigenschaft, den Zusammenhalt des Systems zu gewährleisten. Sie besteht aus einem Grundpolymer und gegebenenfalls den üblichen Zusätzen. Die Auswahl des Grundpolymers richtet sich nach den chemischen und physikalischen Eigenschaften des Physostigmins. Beispielhafte Polymere sind Kautschuk, kautschukähnliche, synthetische Homo-, Co- oder Blockpolymere, Polyacrylsäureester und deren Copolymeren. Grundsätzlich kommen alle Polymere in Frage, die bei der Herstellung von Haftklebern eingesetzt werden. Physiologisch unbedenklich sind, und Physostigmin nicht zersetzen. Besonders bevorzugt sind solche, die aus Blockcopolymeren auf Basis von Styrol und 1,3-Dienen, Polyisobutylenen oder Polymeren aus Acrylat- und/oder Methacrylat bestehen. Von den Blockcopolymeren auf Basis von Styrol und 1,3-Dienen werden ganz besonders lineare Styrol-Isopren-Blockcopolymere eingesetzt.

Als Polymere auf Acrylatbasis werden Acrylatcopolymere aus 2-Ethylhexylacrylat, Vinylacetat und Acrylsäure mit bzw. ohne Titanchelatester bevorzugt. Als Methacrylate werden Copolymere auf Basis von Dimethylaminoethylmethacrylate und neutralen Methacrylsäureestern bevorzugt. Als Zusätze werden Ester von hydriertem Kolophonium, vorzugsweise insbesondere dessen Methyl- und Glycerinester verwendet.

Die Art der möglichen übrigen Zusätze hängt vom eingesetzten Polymer und dem Wirkstoff ab: Nach ihrer Funktion lassen sie sich einteilen in Weichmacher, Klebrigmacher, Stabilisatoren, Trägerstoffe, diffusions- und penetrationsregulierende Zusätze oder Füllstoffe. Die hierfür in Frage kommenden physiologisch unbedenklichen Substanzen sind dem Fachmann bekannt.

Die Reservoirschicht besitzt eine solche Eigenklebrigkeit, daß ein dauernder Kontakt zur Haut sichergestellt ist.

Beispiele für geeignete Weichmacher sind Diester von Dicarbonsäuren, z.B. Di-n-butyladipat sowie Triglyceride, insbesondere mittelkettige Triglyceride der Capryl/Caprinsäure des Kokosöls zu nennen.

Zwischen der Matrix (1) und der Deckschicht (4) ruht das Wirkstoffdepot (2). Es besteht aus einem textilen Flächengebilde und der Physostigmin-Lösung. Das textile Flächengebilde kann eine Ronde aus einem Vlies-Stoff (Fasergemisch, Zellwolle/Baumwolle, 50:50 mit einem Flächengewicht von 40 $g/m^2$, bzw. 80 $g/m^2$) mit einem Durchmesser von 20 bis 50 mm sein. Andere Textile und andere Durchmesser sind möglich.

Als Verbindung mit mindestens einer sauren Gruppe werden höhere Fettsäuren bevorzugt, da das Ausmaß möglicher Hautreizungen, hervorgerufen durch migrierende Säuren, abhängig ist von der Kettenlänge der Säuren. Beispiele für geeignete Säuren sind Ölsäure, Isostearinsäure, Undecensäure und Versaticsäure.

4

Die ablösbare Schutzschicht, die mit der Reservoirschicht in Berührung steht, und vor der Anwendung entfernt wird, besteht beispielsweise aus den selben Materialien, wie sie zur Herstellung der Deckschicht (4) benutzt werden, vorausgesetzt, daß sie ablösbar gemacht werden, wie z.B. durch eine Silikonbehandlung. Andere ablösbare Schutzschichten sind z.B. Polytetrafluorethylen, behandeltes Papier, Celluphan, Polyvinylchlorid u.ä. Wird das erfindungsgemäße Laminat vor Aufbringen der Schutzschicht in therapiegerechte Formate (Pflaster) aufgeteilt, so können die dann aufzubringenden Schutzschichtformate ein überstehendes Ende aufweisen, mit dessen Hilfe sie leichter von dem Pflaster abgezogen werden können.

Im folgenden soll die Fertigung erläutert werden:

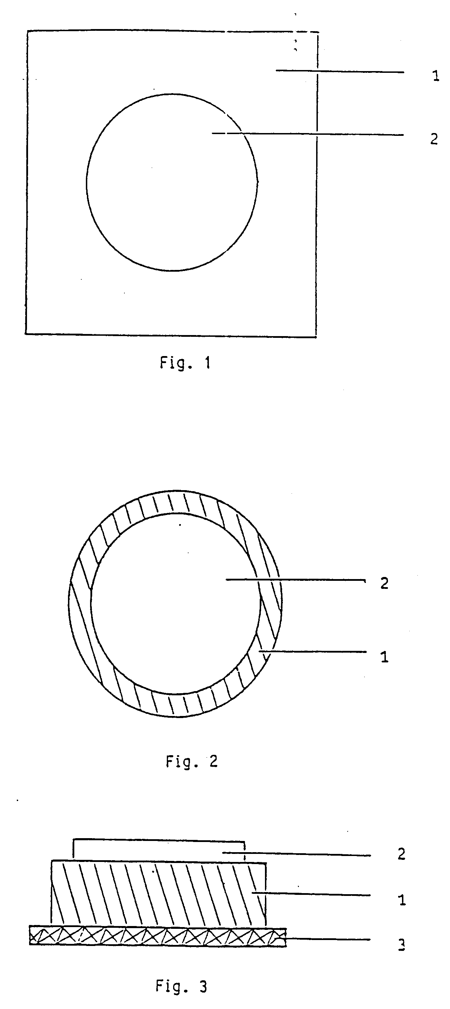

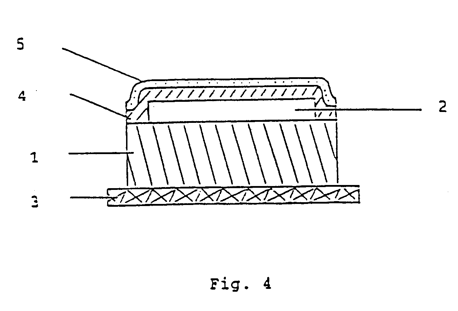

Zunächst wird die Matrix (1) hergestellt, deren Zusammensetzung bei den einzelnen Rezepturbeispielen angegeben ist. Nach Entfernen einer Abdeckfolie wird ein rundes Vlies aufgelegt, dessen Fläche 13,72 cm$^2$ beträgt. Als Vlies wird ein Vliesstoff-Fasergemisch Zellwolle/Baumwolle 70:30, Flächengewicht 40 g pro m$^2$ benutzt. Anschließend schneidet man aus der mit dem Vlies belegten Matrix eine Fläche von 56 cm$^2$ rechteckig aus. Vlies und Matrix sind in Figur 1 und Figur 2 dargestellt. Mit einer geeigneten Vorrichtung, z.B. einer Tropfpipette, wird Physostigmin, 25 %ig in Ölsäure oder 35 %ig in Undecensäure gelöst, auf das Vlies aufgebracht. Die genauen Mengen sind in den einzelnen Rezepturbeispielen angegeben.

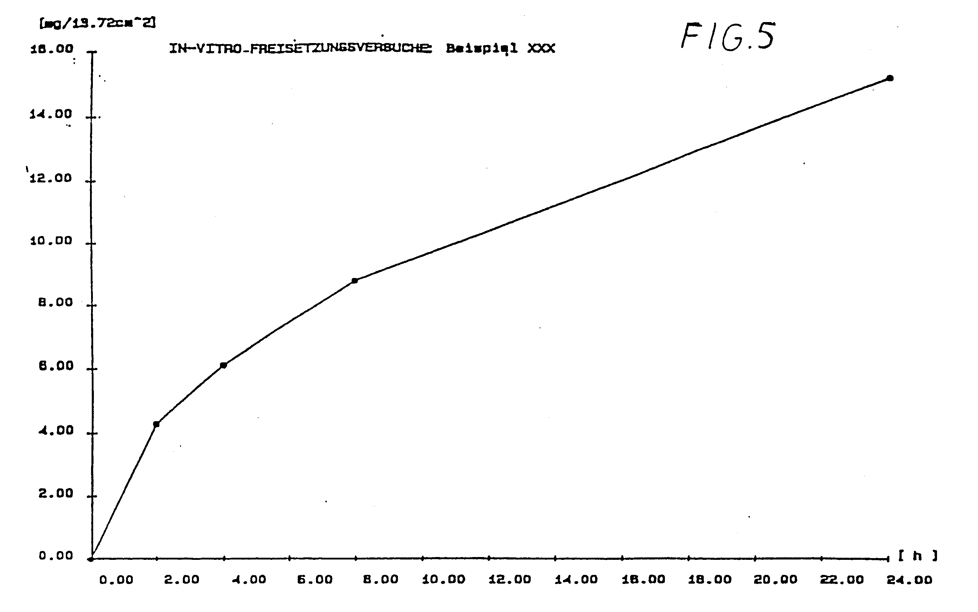

Nach dem Auftragen werden Wirkstoffdepot (2) und Matrix (1) mit einer Deckschicht (4) abgedeckt. Die Deckschicht ist ein Klebestrich, der aus einem Multiacrylcopolymer besteht, der auf eine Polyester-Folie 15 µ aufgestrichen wurde. Nach Entfernen des Lösemittels beträgt das Flächengewicht 30 g/m$^2$. Nach dem Abdecken wird eine runde Fläche von 21,23 cm$^2$ ausgestanzt, so daß das wirkstofflösunggetränkte Vlies in der Mitte ruht (Figuren 3 und 4). Anschließend werden die Ränder abgegittert und das transdermale therapeutische System 14 Tage lang bei 40°C gelagert.

Die Entstehung des erfindungsgemäßen Systems ist in den Abbildungen Figuren 1 bis 4 dargestellt. Figur 1 zeigt in Aussicht das Vlies (2), das auf die Matrix (1) aufgelegt wurde. Nach dem Auflegen des Vlieses (2) auf die Matrix (1) wird die flüssige Wirkstoffzubereitung aufgebracht, wodurch das Wirkstoffdepot (2) entsteht. Anschließend wird die Deckschicht (4) über Matrix (1) und über Wirkstoffdepot (2) kaschiert. Dann wird ausgestanzt. Die Ränder werden durch Abgittern entfernt. Die Figuren 2 und 3 zeigen schematisch in Aufsicht (Figur 2) und im Querschnitt (Figur 3), wie das Wirkstoffdepot (2) nach dem Abgittern auf der Matrix (1) und der Endabdeckung (3) ruht.

Vollständig ist das System in Figur 4 im Querschnitt dargestellt. In den Figuren 1 bis 4 bedeuten:

1    Matrix
2    Wirkstoffdepot bzw. Vlies
3    Schutzschicht
4    Deckschicht
5    Metallfolie

Rezepturbeispiele:

Beispiel I:

Fertigen der Matrix: 10,91 kg einer 40%igen Lösung eines selbstvernetzendes Acrylatcopolymeren aus 2-Ethylhexylacrylat, Vinylacetat und Acrylsäure mit Titanchelatester, Lösungsmittelgemisch: Ethylacetat, Ethanol, Heptan, Methanol 64:25:9:2 und 2,4 kg einer 50 %igen Lösung eines kationischen Copolymerisats auf Basis von Dimethylaminoethylmethacrylat und neutralen Methacrylsäureestern in Ethylacetat werden mit 360 g Triglyceriden der Capryl/Caprinsäuren unter Rühren bei Raumtemperaturen gemischt. Mit einem geeigneten Rakel streicht man auf eine alubedampfte Polyesterfolie auf und deckt mit einer LDPE-Folie, ab. Das Flächengewicht soll zwischen 260,0 g und 275 g/m$^2$ liegen.

| Zusammensetzung von Matrix 1: | |
| --- | --- |
| selbstvernetzendes Acrylatcopolymer ACP$_1$ | 74 % |
| Methacrylatcopolymer MCP | 20 % |
| Triglyceride der Capryl/Caprinsäuren (TriG) | 6 % |
| | 100 % |

Die LDPE-Folie wird abgezogen und das Vlies wird aufgelegt. Auf das Vlies wird eine Mischung von 12 mg Physostigmin und 36 mg Ölsäure mit einer Tropfpipette aufgetragen.

Alle weiteren Beispiele und Beispiel I sind in den folgenden Tabellen aufgeführt. Die in vitro-Freisetzung wurde in einem Schüttelwasserbad bei 37°C bestimmt. Das Akzeptormedium waren 100 ml physiologische Kochsalz-Lösung, die nach 2, 4 und 8 Stunden komplett ausgewechselt wurden. Die Konzentrationen wurden nach 2, 4, 8 und 24 Stunden per UV-Spektroskopie bestimmt.

Die in Tabellen aufgeführten Freisetzungswerte sind jeweils die Summen der gemessenen Konzentrationen.

Die Tabelle 1 zeigt den Aufbau und die in vitro-Freisetzung der Beispiele I - IV. Bei diesen Beispielen wurde jeweils die gleiche Matrix benutzt, die Auftragsmenge aber variiert; aufgetragen wurde Physostigmin, 25 %ig in Ölsäure gelöst.

Tabelle 1

| Beispiel | Physostigmin | FG | Freisetzung (mg) | | | (%) | |
|---|---|---|---|---|---|---|---|
| | | | 2 h | 4 h | 8 h | 24 h | 24 h |
| I | 12 mg | 260 | 1,64 | 2,28 | 3,17 | 5,62 | 46,8 |
| II | 12 mg | 180 | 3,28 | 4,60 | 6,29 | 9,19 | 76,6 |
| III | 16 mg | 260 | 2,77 | 4,03 | 6,71 | 10,38 | 56,1 |
| IV | 20 mg | 180 | 6,43 | 9,15 | 12,76 | 17,47 | 87,4 |
| FG = Flächengewicht der Matrices (g/m$^2$) | | | | | | | |

Man erkennt, daß mit erhöhtem Physostigmin-Anteil nicht nur die absolute, sondern auch die relative Freisetzung ansteigt.

Bei den Beispielen IV - XXIII wurden jeweils 20 mg Physostigmin - in 60 mg Ölsäure gelöst - zudosiert. Variiert wurden die prozentualen Zusammensetzungen der Matrices und die Flächengewichte (FG). Die Angaben zu den Matrices sind in der Tabelle 2 wiedergegeben.

In den Tabellen bedeuten die Abkürzungen:

ACP$_1$: selbstvernetzendes Acrylatcopolymeres aus 2-Ethylhexylacrylat, Vinylacetat, Acrylsäure mit Titanchelatester als Vernetzer

ACP$_2$: nicht selbstvernetzendes Acrylatcopolymeres aus 2-Ethylhexylacrylat, Vinylacetat und Acrylsäure

NCP: Copolymerisat auf Basis von Dimethylaminoethylmethacrylat und neutralen Methacrylsäureestern

TriG: Triglyceride der Capryl/Caprinsäure

K.E.: Ester der Harzsäure Kolophonium mit Glycerin

## Tabelle 2

Tabellarische Zusammensetzung der Matrices und in vitro-Freisetzung

| Beispiele | ACP | NCP | TriG | Zusätze | FG | Freisetzung [mg] | | | | Bemerkungen |
|---|---|---|---|---|---|---|---|---|---|---|
| | | | | | | 2 h | 4 h | 8 h | 24 h | |
| IV | $ACP_1$:74 % | 20 % | 6 % | – | 180g/m² | 6,43 | 9,15 | 12,76 | 17,47 | |
| V | $ACP_1$:76 % | 20 % | 4 % | – | 320g/m² | 3,58 | 5,06 | 7,14 | 12,16 | |
| VI | $ACP_1$:78 % | 20 % | 2 % | – | 210g/m² | 5,65 | 8,07 | 11,42 | 17,34 | |
| VII | $ACP_1$:78 % | 20 % | 2 % | – | 310g/m² | 3,92 | 5,57 | 7,86 | 13,32 | |
| VIII | $ACP_1$:80 % | 20 % | – | – | 210g/m² | 4,76 | 6,93 | 10,02 | 16,54 | |
| IX | $ACP_1$:80 % | 20 % | – | – | 310g/m² | 4,15 | 5,89 | 8,31 | 13,81 | |
| X | $ACP_1$:86 % | 10 % | 4 % | – | 295g/m² | 3,06 | 4,41 | 6,39 | 10,82 | |
| XI | $ACP_1$:68,5 | 20 % | 4 % | K.E. :7,5% | 320g/m² | 3,16 | 4,45 | 6,31 | 10,40 | |
| XII | $ACP_1$:66 % | 20 % | 4 % | K.E. :10 % | 320g/m² | 3,11 | 4,35 | 6,20 | 10,19 | |
| XIII | $ACP_1$:76 % | 10 % | 4 % | K.E. :10 % | 310g/m² | 2,58 | 3,67 | 5,32 | 8,92 | |
| XIV | $ACP_2$:56 % | 30 % | 4 % | Ölsäure:10 % | 240g/m² | 6,35 | 9,49 | 14,91 | 19,89 | |
| XV | $ACP_2$:56 % | 30 % | 4 % | Ölsäure:10 % | 340g/m² | 3,70 | 5,48 | 7,77 | 13,43 | |
| XVI | $ACP_2$:46 % | 40 % | 4 % | Ölsäure:10 % | 240g/m² | 5,71 | 8,55 | 12,38 | 17,74 | |

EP 0 377 147 B1

| Beispiele | ACP | NCP | TriG | Zusätze | FG | Freisetzung [mg] | | | | Bemerkungen |
|---|---|---|---|---|---|---|---|---|---|---|
| | | | | | | 2 h | 4 h | 8 h | 24 h | |
| XVII | $ACP_2$:46 % | 40 % | 4 % | Ölsäure:10 % | 340g/m² | 2,99 | 4,46 | 6,36 | 11,59 | |
| XVIII | $ACP_1$:59 % | 20 % | 6 % | Ölsäure: 5 % | 230g/m² | 6,95 | 10,22 | 13,88 | 18,22 | |
| XIX | $ACP_1$:69 % | 20 % | 6 % | Ölsäure: 5 % | 345g/m² | 4,44 | 6,58 | 9,17 | 14,62 | |
| XX | $ACP_2$:74 % | 20 % | 6 % | — | 195g/m² | 5,53 | 8,17 | 11,45 | 17,04 | siehe IV |
| XXI | $ACP_2$:74 % | 20 % | 6 % | — | 290g/m² | 3,23 | 4,77 | 6,69 | 11,26 | |
| XXII | $ACP_2$:59 % | 20 % | 6 % | Ölsäure:5 % | 240g/m² | 5,73 | 8,47 | 11,80 | 17,17 | siehe XVIII |
| XXIII | $ACP_2$:69 % | 20 % | 6 % | Ölsäure:5 % | 350g/m² | 3,55 | 5,22 | 7,32 | 12,31 | |

Bei den Beispielen XXIV - XXXI wurden jeweils 20 mg Physostigmin - mit 37 mg Undecensäure vermischt - zudosiert. Variiert wurden die prozentualen Zusammensetzungen der Matrices und die Flächengewichte; die Angaben zu den Matrices sind in der Tabelle 3 wiedergegeben.

8

Tabelle 3

| Beispiele | ACP | NCP | TriG | Zusätze | FG | Freisetzung [mg] | | | | Bemerkungen |
|---|---|---|---|---|---|---|---|---|---|---|
| | | | | | | 2 h | 4 h | 8 h | 24 h | |
| XXIV | ACP 74 % | 20 % | 6 % | – | 200g/m² | 7,05 | 10,23 | 14,9 | 19,57 | siehe IV |
| XXV | ACP 74 % | 20 % | 6 % | – | 295g/m² | 4,48 | 6,48 | 9,32 | 15,84 | |
| XXVI | ACP 80 % | 20 % | – | – | 195g/m² | 5,97 | 8,89 | 13,16 | 19,13 | siehe VIII |
| XXVII | ACP 66 % | 20 % | – | – | 295g/m² | 4,00 | 5,92 | 8,7 | 15,24 | siehe IX |
| XXVIII | ACP 66 % | 20 % | 4 % | K.E. :10 % | 210g/m² | 5,88 | 8,84 | 13,33 | 18,94 | |
| XXIX | ACP 66 % | 20 % | 4 % | K.E. ·10 % | 305g/m² | 3,80 | 5,60 | 8,31 | 15,06 | siehe XII |
| XXX | ACP 86 % | 10 % | 4 % | – | 200g/m² | 4,65 | 6,72 | 9,61 | 15,90 | |
| XXXI | ACP 86 % | 10 % | 4 % | – | 295g/m² | 3,20 | 4,64 | 6,69 | 11,73 | siehe X |

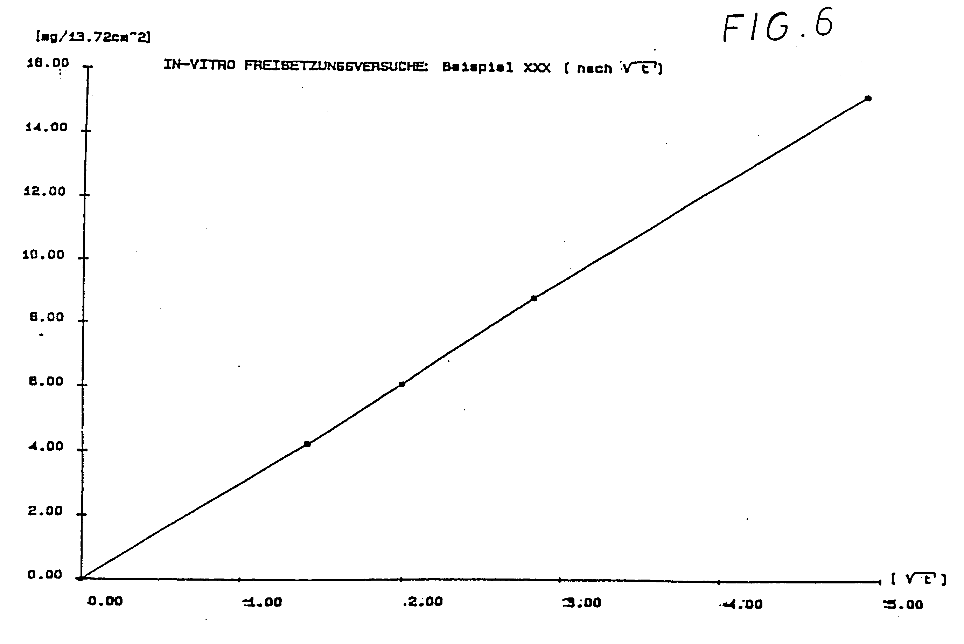

Die Figuren 5 und 6 zeigen das in vitro-Freisetzungsverhalten des Beispiels XXX, das wahllos herausgegriffen wurde. Da die freigesetzte Menge gegen die Wurzel der Zeit aufgetragen - wie bei fast allen Beispielen - eine Gerade ergibt, kann gezeigt werden, daß Systeme entwickelt wurden, die matrixgesteuert den Wirkstoff kontrolliert abgeben, da die Forderungen des Modells von Higuchi eindeutig erfüllt werden.

Es zeigt sich, daß die Freisetzung vom molaren Verhältnis der Carbonsäuren zum basischen Polymer bestimmt werden. Es kann gezeigt werden, daß die Freisetzung um so höher ist, je weniger Säure und je mehr basischer Polymer eingearbeitet wurde. Die Freisetzung ist um so höher, je weniger undisoziierte Säure nach der Lagerung im System vorhanden ist; da in molaren Mengen weniger Undecensäure verwendet wurde, als Ölsäure, ist auch verständlich, warum bei gleicher Matrix die Undecensäuremuster mehr Physostigmin freisetzen als die Ölsäuremuster. Durch die Abstufungen des Weichmachers kann demonstriert werden, daß der Weichmacher keinen Einfluß auf die in vitro-Freisetzung hat.

**Patentansprüche**

**Patentansprüche für folgende Vertragsstaaten : AT, BE, CH, DE, FR, GB, IT, LI, LU, NL, SE**

1. Transdermales therapeutisches System zur Verabreichung von Physostigmin an die Haut aus einer wirkstoffundurchlässigen Deckschicht (4), einer haftklebenden Reservoirschicht (1) und ggf. einer wiederablösbaren Schutzschicht, dadurch gekennzeichnet, daß die Reservoirschicht eine Matrix (1) ist, die 10 bis 90 Gew.-% eines Polymermaterials, ausgewählt aus den Gruppen, bestehend aus Blockpolymeren auf Basis von Styrol und 1,3-Dienen, Polyisobutylenen, Polymeren auf Acrylat- und/oder Methacrylatbasis, wobei in die Matrix ein hoher Anteil eines basischen Polymers eingearbeitet ist, ggf. Ester hydrierten Kolophoniums, 0 bis 30 Gew.-% Weichmacher auf Basis von Kohlenwasserstoffen und/oder Estern enthält, und daß auf die Matrix ein Wirkstoffdepot (2) aufgebracht ist, welches Physostigmin in hochprozentiger Form, d.h. mindestens 25%-ig in Lösung mit einer mehr als 10 Kohlenstoffatome besitzenden Carbonsäure und somit unmittelbar nach der Fertigung 15 bis 85 Gew.-% einer Physostigminlösung enthält.

2. Transdermales therapeutisches System nach Anspruch 1, dadurch gekennzeichnet, daß das Polymermaterial selbstvernetzendes Acrylatcopolymeres aus 2-Ethylhexylacrylat, Vinylacetat, Acrylsäure und Titanchelatester enthält.

3. Transdermales therapeutisches System nach Anspruch 1, dadurch gekennzeichnet, daß das Polymermaterial nicht selbstvernetzendes Acrylatcopolymeres aus 2-Ethylhexylacrylat, Vinylacetat und Acrylsäure enthält.

4. Transdermales therapeutisches System nach Anspruch 1, dadurch gekennzeichnet, daß das Polymermaterial als Polymer auf Basis von Methacrylaten ein Copolymer auf Basis von Dimethylaminoethylmethacrylat und neutralen Methacrylsäureestern enthält.

5. Transdermales therapeutisches System nach Anspruch 1, dadurch gekennzeichnet, daß die Matrix als Ester des hydrierten Kolophoniums dessen Methylester enthält.

6. Transdermales therapeutisches System nach Anspruch 1, dadurch gekennzeichnet, daß die Matrix als Ester des hydrierten Kolophoniums dessen Glycerinester enthält.

7. Transdermales therapeutisches System nach Anspruch 1, dadurch gekennzeichnet, daß die Reservoirschicht als Weichmacher Dioctylcyclohexan enthält.

8. Transdermales therapeutisches System nach Anspruch 1, dadurch gekennzeichnet, daß die Reservoirschicht als Weichmacher Di-n-butyladipat enthält.

9. Transdermales therapeutisches System nach Anspruch 1, dadurch gekennzeichnet, daß die Reservoirschicht als Weichmacher Triglyceride enthält.

10. Transdermales therapeutisches System nach Anspruch 1, dadurch gekennzeichnet, daß die Reservoirschicht als Weichmacher Isopropylmyristat enthält.

11. Transdermales therapeutisches System nach Anspruch 1, dadurch gekennzeichnet, daß das Lösemittel der Physostigmin-Lösung im Wirkstoffdepot eine Verbindung mit mindestens einer sauren Gruppe ist.

12. Transdermales therapeutisches System nach Anspruch 11, dadurch gekennzeichnet, daß die Verbindung mit mindestens eine sauren Gruppe eine Carbonsäure ist.

**13.** Transdermales therapeutisches System nach Anspruch 12, dadurch gekennzeichnet, daß die Carbonsäure Ölsäure oder Undecensäure umfasst.

**14.** Transdermales therapeutisches System nach Anspruch 12, dadurch gekennzeichnet, daß die Carbonsäure ein Gemisch aus Octadecansäuren umfasst.

**15.** Transdermales therapeutisches System nach Anspruch 12, dadurch gekennzeichnet, daß die Carbonsäure Versaticsäuren umfasst.

**16.** Verfahren zur Herstellung eines transdermalen therapeutischen Systems nach Anspruch 1, dadurch gekennzeichnet, daß das Physostigmin oder dessen pharmazeutisch akzeptables Salz in Lösung auf ein textiles Flächenmaterial, das auf einer vorgefertigten selbstklebenden gegebenenfalls mit einer ablösbaren Schutzschicht versehenen Matrix ruht, aufgebracht wird, wonach Matrix und Wirkstoffdepot mit einer Deckschicht versehen werden.

**Patentansprüche für folgende Vertragsstaaten : ES, GR**

**1.** Verfahren zur Herstellung eines transdermalen therapeutischen Systems zur Verabreichung von Physostigmin an die Haut enthaltend eine wirkstoffundurchlässige Deckschicht, eine haftklebende Reservoirschicht und gegebenenfalls eine wiederablösbare Schutzschicht, dadurch gekennzeichnet, daß auf einer vorgefertigten, selbstklebenden, gegebenenfalls mit einer ablösbaren Schutzschicht versehenen Matrixschicht ein textiles Flächengebilde angeordnet wird, auf das textile Flächengebilde das Physostigmin oder eines seiner pharmazeutisch akzeptablen Salze in Lösung unter Bildung eines Wirkstoffdepots aufgebracht wird, wonach Matrix und Wirkstoffdepot mit einer Deckschicht versehen werden.

**2.** Verfahren nach Anspruch 1, dadurch gekennzeichnet, daß das transdermale therapeutische System nach seiner Fertigung 14 Tage gelagert wird, wobei Physostigmin aus dem Wirkstoffdepot in die Matrix übertritt, so daß die Matrix zur Reservoirschicht wird.

**3.** Verfahren nach Anspruch 1, dadurch gekennzeichnet, daß die Reservoirschicht eine Matrix (1) ist, die 10 - 90 Gew-% eines Polymermaterials, ausgewählt aus den Gruppen bestehend aus Blockcopolymeren auf Basis von Styrol und 1,3-Dienen, Polyisobutylenen, Polymeren auf Acrylat- und/oder Methacrylatbasis wobei in die Matrix ein hoher Anteil eines basischen Polymers eingearbeitet ist, gegebenenfalls Ester hydrierten Kolophoniums, 0- 30 Gew.-% Weichmacher auf Basis von Kohlenwasserstoffen und/oder Estern enthält und daß das Wirkstoffdepot unmittelbar nach der Fertigung 15 bis 85 Gew.-% einer 0,1 bis 70 Gew.-%igen Physostigmin-Lösung enthält.

**4.** Verfahren nach Anspruch 1, dadurch gekennzeichnet, daß das Polymermaterial selbstvernetzendes Acrylatcopolymeres aus 2-Ethylhexylacrylat, Vinylacetat, Acrylsäure und Titanchelatester enthält.

**5.** Verfahren nach Anspruch 1, dadurch gekennzeichnet, daß das Polymermaterial nicht selbstvernetzendes Acrylatcopolymeres aus 2-Ethylhexylacrylat, Vinylacetat und Acrylsäure enthält.

**6.** Verfahren nach Anspruch 1, dadurch gekennzeichnet, daß das Polymermaterial als Polymer auf Basis von Methacrylaten ein Copolymer auf Basis von Dimethylaminoethylmethacrylat und neutralen Methacrylsäureestern enthält.

**7.** Verfahren nach Anspruch 1, dadurch gekennzeichnet, daß die Matrix als Ester des hydrierten Kolophoniums dessen Methylester enthält.

**8.** Verfahren nach Anspruch 1, dadurch gekennzeichnet, daß die Matrix als Ester des hydrierten Kolophoniums dessen Glycerinester enthält.

**9.** Verfahren nach Anspruch 1, dadurch gekennzeichnet, daß die Reservoirschicht als Weichmacher Dioctylcyclohexan enthält.

**10.** Verfahren nach Anspruch 1, dadurch gekennzeichnet, daß die Reservoirschicht als Weichmacher Di-n-butyladipat enthält.

**11.** Verfahren nach Anspruch 1, dadurch gekennzeichnet, daß daß die Reservoirschicht als Weichmacher Triglyceride enthält.

**12.** Verfahren nach Anspruch 1, dadurch gekennzeichnet, daß die Reservoirschicht als Weichmacher Isopropylmyristat enthält.

**13.** Verfahren nach Anspruch 1, dadurch gekennzeichnet, daß das Lösemittel der Physostigmin-Lösung im Wirkstoffdepot eine Verbindung mit mindestens einer sauren Gruppe ist.

**14.** Verfahren nach Anspruch 13, dadurch gekennzeichnet, daß die Verbindung mit mindestens eine sauren Gruppe eine Carbonsäure ist.

**15.** Verfahren nach Anspruch 14, dadurch gekennzeichnet, daß die Carbonsäure Ölsäure oder Undecensäure umfaßt.

**16.** Verfahren nach Anspruch 14, dadurch gekennzeichnet, daß die Carbonsäure ein Gemisch aus Octadecansäuren umfaßt.

**17.** Verfahren nach Anspruch 14, dadurch gekennzeichnet, daß die Carbonsäure Versaticsäuren umfaßt.

**18.** Träger für die Verwendung zur transdermalen Verabreichung von Physostigmin, bestehend aus einer wirkstoffundurchlässigen Rückschicht, einer haftklebenden Schicht sowie gegebenenfalls aus einer wiederablösbaren Schutzschicht, dadurch gekennzeichnet, daß die Reservoirschicht haftklebend ist und 10 - 90 Gew.-% Polymermaterial, 0 - 30 Gew.-% Weichmacher enthält, und daß das Wirkstoffdepot unmittelbar nach der Fertigung 15 - 85 Gew.-% einer 0,1 - 70 Gew.-%igen Physostigmin-Lösung enthält.

**Claims**

**Claims for the following Contracting States : AT, BE, CH, DE, FR, GB, IT, LI, LU, NL, SE**

**1.** A transdermal therapeutical system for the administration of physostigmine to the skin, consisting of a cover layer (4) which is impermeable to active substances, a pressure-sensitive adhesive reservoir layer (1), and optionally a removable protective layer, characterized in that the reservoir layer is a matrix (1) comprising 10-90%-wt of a polymeric material selected from the groups consisting of block copolymers based on styrene and 1,3-dienes, polyisobutylenes, polymers based on acrylate and/or methacrylate, whereby in the matrix there is incorporated a large portion of a basic polymer, optionally esters of hydrogenated colophonium, 0-30%-wt softener based on hydrocarbons and/or esters, and that to the matrix there is applied an active substance depot (2) which contains physostigmine in a form containing a high percentage thereof, i.e. at least 25% in solution with a carboxylic acid having more than 10 carbon atoms, and, thus, immediately after production comprises 15-85%-wt of a physostigmine solution.

**2.** The transdermal therapeutical system according to claim 1, characterized in that the polymeric material comprises self-crosslinking acrylate copolymer of 2-ethylhexyl acrylate, vinyl acetate, acrylic acid and titanium chelate ester.

**3.** The transdermal therapeutical system according to claim 1, characterized in that the polymeric material comprises non-self-crosslinking acrylate copolymer of 2-ethylhexyl acrylate, vinyl acetate, and acrylic acid.

**4.** The transdermal therapeutical system according to claim 1, characterized in that the polymeric material comprises as polymer on the basis of methacrylates a copolymer on the basis of dimethylaminoethyl methacrylate and neutral methacrylic acid esters.

**5.** The transdermal therapeutical system according to claim 1, characterized in that the matrix comprises as ester of the hydrogenated colophonium the methylester thereof.

6. The transdermal therapeutical system according to claim 1, characterized in that the matrix comprises as ester of the hydrogenated colophonium the glycerol ester thereof.

7. The transdermal therapeutical system according to claim 1, characterized in that the reservoir layer comprises as softener dioctylcyclohexane.

8. The transdermal therapeutical system according to claim 1, characterized in that the reservoir layer comprises as softener di-n-butyl adipate.

9. The transdermal therapeutical system according to claim 1, characterized in that the reservoir layer comprises as softeners triglycerides.

10. The transdermal therapeutical system according to claim 1, characterized in that the reservoir layer comprises as softener isopropylmyristate.

11. The transdermal therapeutical system according to claim 1, characterized in that the solvent of the physostigmine solution in the active substance depot is a compound with at least one acidic group.

12. The transdermal therapeutical system according to claim 11, characterized in that the compound comprising at least one acidic group is a carboxylic acid.

13. The transdermal therapeutical system according to claim 12, characterized in that the carboxylic acid comprises oleic acid or undecenoic acid.

14. The transdermal therapeutical system according to claim 12, characterized in that the carboxylic acid comprises a mixture of octadecanoic acids.

15. The transdermal therapeutical system according to claim 12, characterized in that the carboxylic acid comprises versatic acids.

16. A process for the production of the transdermal therapeutical system according to claim 1, characterized in that the physostigmine or its pharmaceutically acceptable salt is applied in solution to a textile fabric material which is positioned on a pre-fabricated self-adhesive matrix optionally being provided with a removable protective layer, and that, thereafter, said matrix and said active substance depot are provided with a cover layer.

**Claims for the following Contracting States : ES, GR**

1. A process for the production of a transdermal therapeutical system for the administration of physostigmine to the skin, consisting of a cover layer (4) which is impermeable to active substances, a pressure-sensitive adhesive reservoir layer (1), and optionally a removable protective layer, characterized in that on a pre-fabricated, self-adhesive matrix layer, which is optionally provided with a removable protective layer, there is positioned a textile fabric, to the textile fabric there is applied the physostigmine or one of the pharmaceutically acceptable salts thereof in solution, thus forming an active substance depot, whereafter the matrix and the active substance depot are provided with a cover layer.

2. The process according to claim 1, characterized in that the transdermal therapeutic system, after its production, is stored for 14 days, whereby physostigmine moves out of the active substance depot into the matrix, so that the matrix turns into the reservoir layer.

3. The process according to claim 1 characterized in that the reservoir layer is a matrix (1) comprising 10-90%-wt of a polymeric material selected from the groups consisting of block copolymers on the basis of styrene and 1,3-dienes, polyisobutylenes, polymers on the basis of acrylate and/or methacrylate, whereby in the matrix there is incorporated a large portion of a basic polymer, optionally esters of hydrogenated colophonium, 0-30%-wt softener on the basis of hydrocarbons and/or esters, and that, immediately after its production, the active substance depot comprises 15-85%-wt of a 0.1-70%-wt physostigmine solution.

**4.** The process according to claim 1, characterized in that the polymeric material comprises self-crosslinking acrylate copolymer of 2-ethylhexyl acrylate, vinyl acetate, acrylic acid and titanium chelate ester.

**5.** The process according to claim 1, characterized in that the polymeric material comprises non-self-crosslinking acrylate copolymer of 2-ethylhexyl acrylate, vinyl acetate, and acrylic acid.

**6.** The process according to claim 1, characterized in that the polymeric material comprises as polymer on the basis of methacrylates a copolymer on the basis of dimethylaminoethyl methacrylate and neutral methacrylic acid esters.

**7.** The process according to claim 1, characterized in that the matrix comprises as ester of the hydrogenated colophonium the methylester thereof.

**8.** The process according to claim 1, characterized in that the matrix comprises as ester of the hydrogenated colophonium the glycerol ester thereof.

**9.** The process according to claim 1, characterized in that the reservoir layer comprises as softener dioctylcyclohexane.

**10.** The process according to claim 1, characterized in that the reservoir layer comprises as softener di-n-butyl adipate.

**11.** The process according to claim 1, characterized in that the reservoir layer comprises as softeners triglycerides.

**12.** The process according to claim 1, characterized in that the reservoir layer comprises as softener isopropylmyristate.

**13.** The process according to claim 1, characterized in that the solvent of the physostigmine solution in the active substance depot is a compound having at least one acidic group.

**14.** The process according to claim 13, characterized in that the compound having at least one acidic group is a carboxylic acid.

**15.** The process according to claim 14, characterized in that the carboxylic acid comprises oleic acid or undecenoic acid.

**16.** The process according to claim 14, characterized in that the carboxylic acid comprises a mixture of octadecanoic acids.

**17.** The process according to claim 14, characterized in that the carboxylic acid comprises versatic acids.

**18.** Carrier to be used for the transdermal application of physostigmine, consisting of a backing layer impermeable to active substance, a pressure-sensitive adhesive layer and optionally a removable protective layer, characterized in that the reservoir layer is pressure-sensitive adhesive and contains 10-90%-wt polymeric material and 0-30%-wt softener, and that the active substance depot, immediately after its production, contains 15-85%-wt of a 0.1-70%-wt physostigmine solution.

**Revendications**
**Revendications pour les Etats contractants suivants : AT, BE, CH, DE, FR, GB, IT, LI, LU, NL, SE**

**1.** Système thérapeutique, transdermique, pour l'administration de physostigmine à la peau, constitué d'une couche de couverture (4) imperméable au principe actif, d'une couche réservoir collant par contact (1) et éventuellement d'une couche de protection amovible, caractérisé en ce que la couche réservoir est une matrice (1) qui contient 10 à 90% en poids d'une matière polymérique, choisie parmi les groupes constitués de polymères séquencés à base de styrène et de 1,3-diènes, de polyisobutylènes, de polymères à base d'acrylate et/ou de méthacrylate, où une proportion élevée d'un polymère basique est incorporée à la matrice, éventuellement d'esters de la colophane hydrogénée, de 0 à 30% en poids de plastifiants à base d'hydrocarbures et/ou d'esters et en ce que, sur la matrice, est appliquée un dépôt (2) de principe actif, qui contient de la physostigmine sous une forme à haut pourcentage, c'est-à-dire à raison d'au moins 25%, en solution avec un acide carboxylique possédant plus de 10 atomes de carbone et qui contient, de ce fait, immédiatement après l'apprêtage, 15 à 85% en poids d'une solution de physostigmine.

2. Système transdermique, thérapeutique, suivant la revendication 1, caractérisé en ce que la matière polymérique contient un copolymère d'acrylate autoréticulant, constitué d'acrylate de 2-éthylhexyle, d'acétate de vinyle, d'acide acrylique et d'ester chélate de titane.

3. Système transdermique, thérapeutique, suivant la revendication 1, caractérisé en ce que la matière polymérique contient un copolymère d'acrylate non réticulant, constitué d'acrylate de 2-éthylhexyle, d'acétate de vinyle et d'acide acrylique.

4. Système transdermique, thérapeutique, suivant la revendication 1, caractérisé en ce que la matière polymérique contient, à titre de polymère à base de méthacrylates, un copolymère à base de méthacrylate de diméthylaminoéthyle et d'esters de l'acide méthacrylique neutres.

5. Système transdermique, thérapeutique, suivant la revendication 1, caractérisé en ce que la matrice contient, à titre d'ester de la colophane hydrogénée, son ester méthylique.

6. Système transdermique, thérapeutique, suivant la revendication 1, caractérisé en ce que la matrice contient, à titre d'ester de la colophane hydrogénée, son ester avec la glycérine.

7. Système transdermique, thérapeutique, suivant la revendication 1, caractérisé en ce que la couche réservoir contient du dioctylcyclohexane à titre de plastifiant.

8. Système transdermique, thérapeutique, suivant la revendication 1, caractérisé en ce que la couche réservoir contient de l'adipate de di-n-butyle à titre de plastifiant.

9. Système transdermique, thérapeutique, suivant la revendication 1, caractérisé en ce que la couche réservoir contient des triglycérides à titre de plastifiants.

10. Système transdermique, thérapeutique, suivant la revendication 1, caractérisé en ce que la couche réservoir contient du myristate d'isopropyle à titre de plastifiant.

11. Système transdermique, thérapeutique, suivant la revendication 1, caractérisé en ce que le solvant de la solution de physostigmine dans le dépôt de principe actif est un composé avec au moins un radical acide.

12. Système thérapeutique, transdermique, suivant la revendication 11, caractérisé en ce que le composé avec au moins un radical acide est un acide carboxylique.

13. Système thérapeutique, transdermique, suivant la revendication 12, caractérisé en ce que l'acide carboxylique est l'acide oléique ou l'acide undécénoïque.

14. Système thérapeutique, transdermique, suivant la revendication 12, caractérisé en ce que l'acide carboxylique est un mélange d'acides octadécanoïques.

15. Système thérapeutique, transdermique, suivant la revendication 12, caractérisé en ce que l'acide carboxylique est constitué d'acides versatiques.

16. Procédé de préparation d'un système thérapeutique, transdermique, suivant la revendication 1, caractérisé en ce que l'on applique la physostigmine ou son sel pharmaceutiquement acceptable, en solution, sur une matière textile plate ou plane qui repose sur une matrice autocollante, préalablement apprêtée, éventuellement pourvue d'une couche de protection amovible, puis on pourvoit la matrice et le dépôt de principe actif d'une couche de couverture.

**EP 0 377 147 B1**

**Revendications pour les Etats contractants suivants : ES, GR**

1. Procédé de préparation d'un système thérapeutique, transdermique, destiné à l'administration de physostigmine à la peau, comprenant une couche de couverture imperméable au principe actif, une couche réservoir collant par contact et, éventuellement, une couche de protection réenlevable ou amovible, caractérisé en ce que sur une couche formant matrice, préalablement apprêtée, autocollante, éventuellement pourvue d'une couche de protection amovible, on agence un article textile plat, sur cet article textile plat, on dépose la physostigmine ou un sel pharmaceutiquement acceptable de celle-ci, en solution, avec formation d'un dépôt de principe actif, puis on pourvoit matrice et dépôt de principe actif d'une couche de protection.

2. Procédé suivant la revendication 1, caractérisé en ce que le système thérapeutique, transdermique, est conservé pendant 14 jours, après son apprêtage, si bien que la physostigmine passe du dépôt de principe actif dans la matrice, en sorte que la matrice devient une couche réservoir.

3. Procédé suivant la revendication 1, caractérisé en ce que la couche réservoir est une matrice (1) qui contient 10 à 90% en poids, d'une matière polymérique, choisie dans les groupes formés de copolymères séquencés à base de styrène et de 1,3-diènes, de polyisobutylènes, de polymères à base d'acrylate et/ou de méthacrylate, où une forte proportion d'un polymère basique est incorporée dans la matrice, éventuellement d'ester de la colophane hydrogénée, de 0 à 30% en poids de plastifiants à base d'hydrocarbures et/ou d'esters et en ce que le dépôt de principe actif contient, immédiatement après l'apprêtage, de 15 à 85% en poids d'une solution à 0,1 à 70% en poids de physostigmine.

4. Procédé suivant la revendication 1, caractérisé en ce que la matière polymérique contient un copolymère d'acrylate autoréticulant, constitué d'acrylate de 2-éthylhexyle, d'acétate de vinyle, d'acide acrylique et d'ester chélate de titane.

5. Procédé suivant la revendication 1, caractérisé en ce que la matière polymérique contient un copolymère d'acrylate non réticulant, constitué d'acrylate de 2-éthylhexyle, d'acétate de vinyle et d'acide acrylique.

6. Procédé suivant la revendication 1, caractérisé en ce que la matière polymérique contient, à titre de polymère à base de méthacrylates, un copolymère à base de méthacrylate de diméthylaminoéthyle et d'esters de l'acide méthacrylique neutres.

7. Procédé suivant la revendication 1, caractérisé en ce que la matrice contient, à titre d'ester de la colophane hydrogénée, son ester méthylique.

8. Procédé suivant la revendication 1, caractérisé en ce que la matrice contient, à titre d'ester de la colophane hydrogénée, son ester avec la glycérine.

9. Procédé suivant la revendication 1, caractérisé en ce que la couche réservoir contient du dioctylcyclohexane à titre de plastifiant.

10. Procédé suivant la revendication 1, caractérisé en ce que la couche réservoir contient de l'adipate de di-n-butyle à titre de plastifiant.

11. Procédé suivant la revendication 1, caractérisé en ce que la couche réservoir contient des triglycérides à titre de plastifiants.

12. Procédé suivant la revendication 1, caractérisé en ce que la couche réservoir contient du myristate d'isopropyle à titre de plastifiant.

13. Procédé suivant la revendication 1, caractérisé en ce que le solvant de la solution de physostigmine dans le dépôt de principe actif est un composé avec au moins un radical acide.

**14.** Procédé suivant la revendication 13, caractérisé en ce que le composé avec au moins un radical acide est un acide carboxylique.

**15.** Procédé suivant la revendication 14, caractérisé en ce que l'acide carboxylique est l'acide oléique ou l'acide undécénoïque.

**16.** Procédé suivant la revendication 14, caractérisé en ce que l'acide carboxylique est un mélange d'acides octadécanoïques.

**17.** Procédé suivant la revendication 14, caractérisé en ce que l'acide carboxylique est constitué d'acides versatiques.

**18.** Support à utiliser pour l'administration transdermique de physostigmine, qui se compose d'une couche dorsale imperméable au principe actif, d'une couche collant par contact, comme éventuellement aussi d'une couche de protection réenlevable ou amovible, caractérisé en ce que la couche réservoir est autocollante et contient de 10 à 90% en poids de matière polymérique, de 0 à 30% en poids de plastifiant et en ce que le dépôt de principe actif contient, immédiatement après l'apprêtage, 15 à 85% en poids d'une solution à 0,1-70% en poids de physostigmine.

Fig. 1

Fig. 2

Fig. 3

Fig. 4

FIG.5

FIG.6

IN-VITRO FREISETZUNGSVERSUCHE: Beispiel XXX ( nach $\sqrt{t}$ )